(19) Europäisches Patentamt
European Patent Office
Office européen des brevets

(11) EP 0 863 769 B1

(12) EUROPÄISCHE PATENTSCHRIFT

(45) Veröffentlichungstag und Bekanntmachung des Hinweises auf die Patenterteilung:
**03.07.2002 Patentblatt 2002/27**

(21) Anmeldenummer: **97948758.4**

(22) Anmeldetag: **01.10.1997**

(51) Int Cl.[7]: **A61K 47/48**

(86) Internationale Anmeldenummer:
**PCT/EP97/05389**

(87) Internationale Veröffentlichungsnummer:
**WO 98/14215 (09.04.1998 Gazette 1998/14)**

(54) **GLYCOKUNJUGATE ALS INHIBITOREN DER VIRALEN ZELLADHÄSION**
GLYCOCONJUGATES AS VIRUS CELL ADHESION INHIBITORS
GLYCONCONJUGUES COMME INHIBITEURS D'ADHERENCE CELLULAIRE VIRALE

(84) Benannte Vertragsstaaten:
**AT BE CH DE FR GB IT LI**

(30) Priorität: **02.10.1996 DE 19640791**

(43) Veröffentlichungstag der Anmeldung:
**16.09.1998 Patentblatt 1998/38**

(73) Patentinhaber: **Syntesome Gesellschaft Fur Med. Biochemie M.B.H.**
**81739 Munchen (DE)**

(72) Erfinder:
- **BOVIN, Nikolai**
  **Moscow, 117871 (RU)**
- **MATROSOVICH, Mikhail**
  **Moscow, 117607 (RU)**
- **TUZIKOV, Alexandr**
  **Moscow, 105037 (RU)**
- **CHINAREV, Alexandr**
  **Moscow, 117871 (RU)**
- **GAMBARYAN, Alexandra**
  **Moscow, 117133 (RU)**
- **ROBERTSON, James, NIBSC**
  **Hertfordshire EN6 3QG (GB)**

(56) Entgegenhaltungen:
**WO-A-94/11005**

- **GAMBARYAN, A. S. ET AL: "Specification of receptor-binding phenotypes of influenza virus isolates from different hosts using synthetic sialylglycopolymers: non-egg-adapted human H1 and H3 influenza A and influenza B viruses share a common high binding affinity for 6'-sialyl (N- acetyllactosamine )" VIROLOGY (1997), 232(2), 345-350 CODEN: VIRLAX;ISSN: 0042-6822, 1997, XP002068479**
- **UNVERZAGT, CARLO ET AL: "Chemical and enzymic synthesis of multivalent sialoglycopeptides" CARBOHYDR. RES. (1994), 251, 285-301 CODEN: CRBRAT;ISSN: 0008-6215, 1994, XP002068480**
- **CHOI, SEOK-KI ET AL: "Generation and in Situ Evaluation of Libraries of Poly(acrylic acid) Presenting Sialosides as Side Chains as Polyvalent Inhibitors of Influenza-Mediated Hemagglutination" J. AM. CHEM. SOC. (1997), 119(18), 4103-4111 CODEN: JACSAT;ISSN: 0002-7863, Mai 1997, XP002068481**
- **CHEMICAL ABSTRACTS, vol. 97, no. 13, 27.September 1982 Columbus, Ohio, US; abstract no. 106070, CORFIELD, ANTHONY P. ET AL: "The specificity of viral sialidases. The use of oligosaccharide substrates to probe enzymic characteristics and strain-specific differences" XP002068482 & EUR. J. BIOCHEM. (1982), 124(3), 521-5 CODEN: EJBCAI;ISSN: 0014-2956, 1982,**
- **CHEMICAL ABSTRACTS, vol. 121, no. 15, 10.Oktober 1994 Columbus, Ohio, US; abstract no. 180061, NISHIMURA, SHINICHIRO ET AL: "Chemoenzymic preparation of a glycoconjugate polymer having a sialyloligosaccharide: Neu5Ac.alpha.(2.fwdarw.3)Gal.beta.(1.fwdar w.4 )GlcNAc" XP002068483 & BIOCHEM. BIOPHYS. RES. COMMUN. (1994), 199(1), 249-54 CODEN: BBRCA9;ISSN: 0006-291X, 1994,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 863 769 B1

Printed by Jouve, 75001 PARIS (FR)

## Beschreibung

Die vorliegende Erfindung betrifft neue multivalente Derivate von Neu5Acα2-6Galβ1-4GlcNAc und die Herstellung dieser Verbindungen. Sie betrifft weiterhin die Verwendung dieser Verbindungen als Inhibitoren der viralen Zelladhäsion nichtadaptierter menschlicher Influenza Viren.

Der erste essentielle Schritt einer viralen Infektion besteht in der Adhäsion eines Virus an die Oberfläche der Wirtszelle. Im Falle von Influenza Viren der Typen A und B, geschieht dies über die Bindung des viralen Hemagglutinins (HA) an terminale Sialyloligosaccharide von Glycoproteinen und Glycolipiden auf der Zelloberfläche der Wirtszelle. Eine mögliche Strategie zur Verhinderung von Influenza Infektionen besteht in der Verwendung von Inhibitoren der Zelladhäsion, diese sollen sich mit den viralen Hemagglutininen verbinden und deren Reaktion mit den Wirtszellen hemmen.

In der Literatur sind bereits eine Reihe von Inhibitoren der Adhäsion von Influenza Viren bekannt. Insbesondere durch die Entwicklung von multivalenten Verbindungen konnten einige Inhibitoren gefunden werden, welche jeweils die Adhäsion eines bestimmten Influenza Stammes in einem in-vitro Test erfolgreich verhindern (siehe Tabelle 2).

Ein Nachteil dieser bekannten Inhibitoren liegt in ihrem engen Wirkungsspektrum, die Verbindung zeigen jeweils nur gegen einen einzigen Stamm hohe Aktivität und sind somit für eine praktische prophylaktische oder therapeutische Anwendungen nur wenig geeignet.

Ein weiterer Nachteil ist, daß die Aktivität dieser bekannten Inhibitoren jeweils nur gegenüber adaptierten Influenza Stämmen gezeigt wurde, welche auf embryonalen Hühnereiern angezogen worden sind. Es ist bekannt, daß die Anzucht menschlicher Influenza Viren auf Hühnereiern zu Aminosäure Mutationen in der Region der Rezeptorbindestelle des Hemagglutinins führt (Robertson, 1993). Die auf diese Weise gefundenen Inhibitoren haben zwar eine hohe Affinität zu den veränderten Hemagglutininen dieser mutierten Viren, während ihre Aktivität gegenüber Wild-Typ Hemagglutin in vielen Fällen geringer ist (siehe polymeres Sialosid BGN-PAA in Tabelle 1).

Weitere bekannte Inhibitoren der viralen Zelladhäsion von Influenza Viren sind bivalente Sialoglycopeptide des Liganden Neu5Acα2-6Galβ1-4GlcNAc (6'-Sialyl-N-acetyllactosamine, 6SLN) (Unverzagt et al, 1994). Der Aufbau dieser Inhibitoren zeichnet sich dadurch aus, daß der Zucker-Ligand 6SLN direkt mit der Polypeptid-Kette des Trägers verbunden ist.

Aufgabe dieser Erfindung war es neue Inhibitoren der viralen Zelladhäsion von menschlichen Influenza Viren zu finden. Diese Inhibitoren sollten ein breites Wirkungsspektrum gegen Influenza Wild-Typ Stämme haben und hierbei eine möglichst hohe inhibitorische Aktivität besitzen.

Es wurde nun gefunden, daß multivalente Konjugate des Sialosidliganden Neu5Acα2-6Galβ1-4GlcNAc (6'-Sialyl-N-acetyllactosamine, 6SLN) neue, hoch aktive Inhibitoren der viralen Zelladhäsion sind.

Gegenstand der Erfindung sind Verbindungen der Formel I

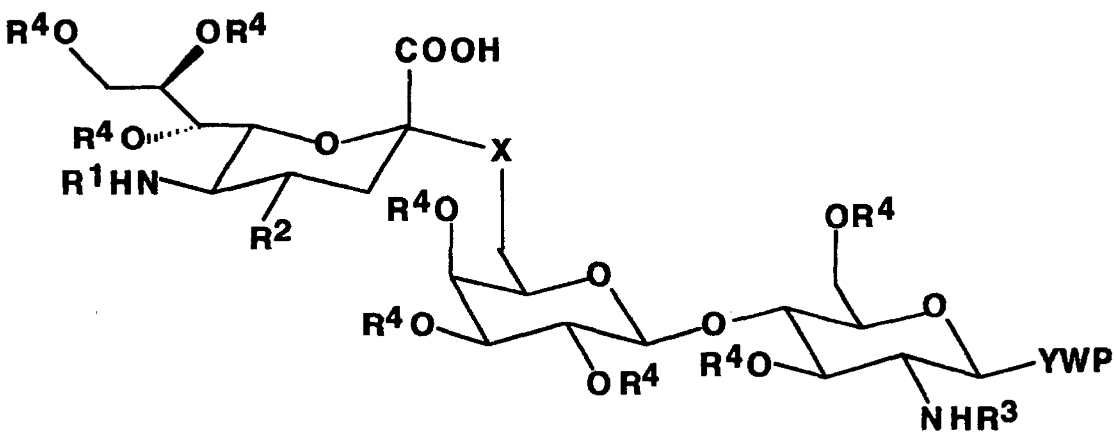

worin $R^1$ eine Acyl oder Thioacylgruppe bedeutet, vorzugsweise eine Acetyl-, Thioacetyl-, Propionyl oder Thiopropionylgruppe ist; $R^2$ H, Hydroxyl, Z-Alkyl, substituiertes Z-Alkyl, Z-Aryl, substituiertes Z-Aryl bedeutet, und Z O, S oder NH entspricht; $R^3$ eine Acyl oder Thioacylgruppe bedeutet; $R^4$ H oder Acyl darstellt; X O, S oder ein lineares $C_1$-$C_4$ Alkyl bedeutet; Y NH, O, S, $CH_2$, oder ein Zucker ist; W ein bifunktioneller Spacer ist;
P ein multivalenter Träger bestehend aus einem der nachfolgenden Stoffe ist: Polyacrylat, Polyacrylamid, N-substituiertes Polyacrylamid, Metacrylamid, N-substituiertes Metacrylamid, Polyacrylsäure, Polycarbonat, Polyester, Polyamid, Polyanhydrid, Polyiminocarbonat, Polyorthoester, Polydioxanon, Polyphosphazen, Polyhydroxycarbonsäure,

Polyaminosäure, Polysaccharid, Protein, Dextran, Chitosan, Glucan, Liposom, Micropartikel.
P kann auch ein niedermolekularer multivalenter Träger wie $(-A_n-NHCH_2)_4C_4$ sein, worin n=3 ist und A eine neutrale oder negativ geladene Aminosäure ist.

Weitere Gegenstände der Erfindung sind:

Verbindungen der Formel I worin P mit Biotin, einem Farbstoff, einem Fluoreszenzfarbstoff oder einem radioaktiven Marker markiert ist.

Die Verwendung der Verbindungen der Formel I zur Bindung an Viren.

Die Verwendung der Verbindungen der Formel I zur Bindung an Influenza Viren.

Die Verwendung der Verbindungen der Formel I zur Bindung an menschliche Influenza Viren.

Die Verwendung der Verbindungen der Formel I worin P mit Biotin, einem Farbstoff, einem Fluoreszenzfarbstoff oder einem radioaktiven Marker markiert ist als Komponenten von Testsystemen zum Screenen von Inhibitoren der viralen Zelladhäsion.

Die Verwendung der Verbindungen der Formel I zur Bindung an Hemagglutinin von Influenza Viren.

Die Verwendung der Verbindungen der Formel I als Inhibitoren der viralen Zelladhäsion an natürliche Rezeptoren.

Die Verwendung der Verbindungen der Formel I zur Bindung an Influenza Viren, an menschliche Influenza Viren, an Hemagglutinin von Influenza Viren und als Inhibitoren der viralen Zelladhäsion an natürliche Rezeptoren jeweils in Kombination mit Neuraminidase-Inhibitoren.

Die Verwendung der Verbindungen der Formel I zur Herstellung von Arzneimitteln für eine prophylaktische oder therapeutische Behandlung viraler Infektionen.

Die Verwendung der Verbindungen der Formel I zur Herstellung von Arzneimitteln für eine prophylaktische oder therapeutische Behandlung viraler Infektionen in Kombination mit Neuraminidase-Inhibitoren.

überaschenderweise zeigte sich auch, daß Verbindungen der Formel I die Zelladhäsion aller bekannter menschlicher Infuenza Stämme der Typen A und B sehr effizient inhibieren. Insbesondere sind diese Inhibitoren aktiv gegen authentische, in der Struktur der Rezeptorbindestelle des Hemagglutins nicht veränderte Influenza Viren. Um eine unveränderte Struktur der Rezeptorbindestelle des Hemagglutinins zu gewährleisten, wurden Influenza Viren aus klinischen Isolaten verwendet, welche ausschließlich auf MDCK-Zellen (Madin-Derby canine kidney cells) angezogen worden sind. Wie ein mittels PCR durchgeführter Vergleich der viralen RNA-Sequenzen auf MDCK gezogener Viren mit den RNA-Sequenzen von Viren aus klinischen Isolaten gezeigt hat, weisen die auf MDCK gezogene Viren keine Mutationen in der Region der Rezeptorbindestelle des Hemagglutinins auf (Robertson, 1993).
Tabelle 1 zeigt einen Vergleich der Inhibition der viralen Zelladhäsion durch die neue Verbindung 6SLN-PAA und durch das Glycokonjugat BGN-PAA, welches bereits früher gegen auf Hühnereiern angezogene Stämme des Typs H3N2 A/Sichuan/1/87 entwickelt wurde. Während die Verbindung 6SLN-PAA ein hervoragender Inhibitor aller authentischer Influenza Stämme ist, hat BGN-PAA ein engeres Wirkungsspektrum, und hat gegen viele Stämme nur eine sehr geringe Aktivität.
Die Vorteile der erfindungsgemäßen Verbindungen der Formel I liegen insbesondere in ihrem Wirkunsspektrum als Inhibitoren der viralen Zelladhäsion aller bekannter menschlicher Influenza Viren der Typen A und B, und in den sich daraus eröffnenden Möglichkeiten diese Verbindungen zu prophylaktischen und therapeutischen Zwecken gegen Influenza Infektionen einzusetzen.
Ein weiterer Vorteil liegt in der leichten Zugänglichkeit von Derivaten der Verbindungen der Formel I, welche einen zusätzlichen Marker tragen, geeignete Marker sind z. B. Biotin, Farbstoffe, Fluoreszenzfarbstoffe oder radioaktive Marker. Diese Derivate können verwendet werden als Komponenten für neue, bisher nicht zugängliche Screening Systeme zur Entdeckung neuer Inhibitoren der viralen Zelladhäsion.

Tabelle 1.

| Inhibition der viralen Zelladhäsion von Influenza Viren durch die polymeren Sialoside BGN-PAA and 6SLN-PAA, gemessen mittels Inhibition der viralen Bindung an Fetuin, wie in Beispiel 8 und in Gambaryan and Matrosovich, 1992 beschrieben.<br>BGN ist Neu5Ac$\alpha$ 2-$OCH_2C_6H_4NHCOCH_2NH$-,<br>6SLN ist Neu5Ac$\alpha$2-6Gal$\beta$1-4GlcNAc$\beta$1-$NHCOCH_2NH$- | | |
|---|---|---|
| **I. Klinische Human-Isolate, vermehrt in MDCK Zellen** | | |
| **Virus** | **Affinität der Bindung an Virus, $K_{aff}$, $\mu$M Neu5Ac** | |
| | **BGN-PAA** | **6SLN-PAA** |
| **H3N2** | | |
| A/NIB/47/89 M | 0.03 | 0.01 |

Tabelle 1. (fortgesetzt)

| I. Klinische Human-Isolate, vermehrt in MDCK Zellen | | |
|---|---|---|
| **Virus** | **Affinität der Bindung an Virus, $K_{aff}$, μM Neu5Ac** | |
| | **BGN-PAA** | **6SLN-PAA** |
| **H3N2** | | |
| A/NIB/3/90 M<br>A/NIB/44/90 M | 0.01<br>0.01 | 0.01<br>0.01 |
| **H1N1** | | |
| A/England/157/83 M<br>A/NIB/12/89 M<br>A/NIB/23/89 M<br>A/NIB/50/89 M | >100<br>>5<br>>5<br>>5 | 0.03<br>0.1<br>0.02<br>0.02 |
| **B** | | |
| B/England/222/82 M<br>B/NIB/48/90 M<br>B/NIB/15/89 M | 0.05<br>0.05<br>0.03 | 0.04<br>0.04<br>0.02 |
| **II.Durch Anzucht in Hühnereiern adaptierte Stämme** | | |
| **H3N2** | | |
| X31 (A/Aichi/2/68)<br>A/England/321/77<br>A/USSR/3/85<br>A/Sichuan/1/87 | 100<br>0.03<br>0.06<br>0.02 | 3<br>0.01<br>0.1<br>0.1 |
| **H2N2** | | |
| A/England/12/64 | 10 | 0.1 |
| **H1N1** | | |
| A/FW/1/50<br>A/USSR/90/77<br>A/Chile/1/83 | 5<br>6<br>30 | 3<br>0.3<br>6 |
| **B** | | |
| B/USSR/100/83<br>B/Ann Arbor/1/86<br>B/Yamagata/11/88 | 5<br>>100<br>>100 | 2<br>3<br>100 |

Die Erfindung wird anhand der Beispiele näher und beispielhaft erläutert.

Beispiel 1.

Herstellung von Neu5Acα2-6Galβ1-4GlcNAcβ1-$NHCOCH_2NH_2$, (modifizierte Methode nach Likhosherstov et al., 1986; und Manger et al., 1992)

Bei Raumtemperatur wurden 13.8 mg 6'-Sialyllactosamin $NH_4^+$-Salz (20 μM, isoliert aus menschlichen Urin) in 1ml gesättigter Ammoniumbicarbonat Lösung gelöst und während 170 h gerührt, während der Inkubation wurde zu der Lösung mehrmals festes Ammoniumbicarbonat bis zur Sättigung zugesetzt. Die Reaktionslösung wurde mit 2ml Wasser verdünnt, eingefroren und lyophilisiert. Man erhielt 13.7 mg Rückstand (war das Gewicht des erhaltenen Produktes größer als die eingesetzte Menge Saccharid, so wurde der Rückstand nochmals in 2ml Wasser aufgenommen und lyophilisiert), dieser wurde in 0.6ml 1 M $NaHCO_3$ aufgenommen. Unter Rührung bei 0°C wurden 34 mg Chloressigsäureanhydrid (200 μM) in 0.34ml Ethylacetat zugegeben. Nach 1 h wurde die Lösung mit Essigsäure neutralisiert und im Vakuum eingeengt. Der erhaltene Rückstand wurde in einer minimalen Menge Wasser aufgelöst und über eine

Sephadex G-25 Säule(1x50 cm) chromatographiert. Die zuckerhaltige Fraktion wurde eingeengt, und der Rückstand in 1 ml gesättigter Ammoniumcarbonat Lösung aufgenommen. Nach 48 h wurde die Lösung mit 2ml Wasser verdünnt, eingefroren und lyophilisiert. Der Rückstand wurde in 2ml 1% Essigsäure aufgelöst und nach 15 h über eine Säule mit 3ml Dowex AG 50W-X4 ($H^+$ Form) gegeben. Die Säule wurde mit 30 ml Wasser gewaschen und dann mit einer 1M Ammoniumhydroxid Lösung eluiert. Nach Einengen des Ammoniumhydroxid Eluats wurden 9.8 mg (67%) des 6'-Sialyllactose N-glycyl Derivates erhalten.

$^1$H-NMR-Spektrum ($D_2O$, δ, ppm): 5.16 (d, 1H, $J_2$ 9 Hz, H-1 Gal), 4.47 (d, 1H, $J_2$ 8 Hz, H-1 GlcNAc), 4.00-3.55 (Gal, GlcNAc, Neu5Ac), 3.46 (s, 2H, COC$\underline{H_2}$NH$_2$), 2.69 (dd, 1H, $J_{3ax}$ 12 Hz, $J_4$ 4.5 Hz, H-3eq Neu5Ac), 2.04 (s, 6H, 2 $CH_3CO$), 1.72 (dd, 1H, $J_4$ 12.5 Hz, H-3ax Neu5Ac).

Der Verlauf der Reaktionen wurde mittels Dünnschichtchromatographie verfolgt (Kieselgel 60, Merck):

| Eluent 1: 2-Propanol/Aceton/Wasser 4:3:2: | |
|---|---|
| 6'SLN | $R_f$ = 0.61 |
| 6'SLN-$NH_2$ | 0.49 Ninhydrin positiv |
| 6'SLN-$NHCOCH_2Cl$ | 0.66 |
| 6'SLN-$NHCOCH_2NH_2$ | 0.09 Ninhydrin positiv |
| Eluent 2: Methanol/Aceton/Wasser 1:1:1: | |
| 6'SLN-$NHCOCH_2NH_2$ | 0.73 Ninhydrin positiv |

Beispiel 2

Herstellung von Neu5Acα2-6Galβ1-4GlcNAcβ1-NHCOCH$_2$NHCO(CH$_2$)$_4$COO-p-C$_6$H$_4$NO$_2$

Bei Raumtemperatur wurden zu einer Lösung von 7.3 mg Neu5Acα2-6Galβ1-4GlcNAcβ1-NHCOCH$_2$NH$_2$ (10 µM) aus Beispiel 1 in 0.2ml DMSO, 39 mg Bis(4-nitrophenyl)-adipat (100 µM) in 0.6ml DMF zugegeben. Nach 3 h wurde das Reaktionsgemisch über eine Sephadex LH-20 Säule (1.8x30 cm, Eluent - Acetonitril/Wasser 1:1) chromatographiert. Die zuckerhaltige Fraktion wurde eingeengt in Wasser aufgenommen, eingefroren und lyophilisiert. Man erhielt 7.4 mg des Glykosids(76%).

Beispiel 3

Herstellung von Tetrakis(N-tert-bulyloxycarbonyl-glycyl-amidomethyl)methan

Ein Gemisch von 1g Tetrakis(aminomethyl)methan (Fleischer et. al., 1971) tetrahydrochlorid (3.6 mmol), 8.52g BocGlyONph (28.8 mmol) und $NEt_3$ (2.5ml, 18†mmol) in 2ml DMF wurde 120 h bei Raumtemperatur gerührt. Das Reaktionsgemisch wurde eingeengt, in 100ml Ethylacetat suspendiert, und mit 2% $H_2SO_4$, Wasser, gesättigter $NaHCO_3$-Lösung und Wasser gewaschen und über $Na_2SO_4$ getrocknet. Die Lösung wurde eingeengt, der Rückstand in Trifluoroethanol gelöst, ein Gemisch $CHCl_3$/EtOAc/MeOH (9:3:1) und $Et_2O$ wurde zugegeben. Man erhielt 2.1 g eines krstallinen Produktes (78%).

DC: $R_f$=0.5 in $CHCl_3$/EtOAc/MeOH/AcOH 9:3:2:0.2 (Ninhydrin-positiv, nach 5 min Behandlung der Platte mit HCI-Gas).

$^1$H-NMR-Spektrum in $D_6$-DMSO (δ, ppm): 7.97 (br t, 1H, CCH$_2$N$\underline{H}$), 7.37 (t, 1H, NH$^{Gly}$), 3,49 (d, 2H, $J_{NH}$ 6 Hz, CH$_2^{Gly}$), 2.76 (br d, 2H, $CCH_2$), 1.37 (s, 9H, $OCMe_3$).

Massenspektrum: 783 (M+Na).

Beispiel 4

Herstellung von Tetrakis(N-tert-butyloxycarbonyl-triglycyl-amidomethyl)methan

Eine Lösung von 0.76g Tetrakis(N-tert-butyloxycarbonyl-glycyl-amidomethyl)-methan (1 mmol) aus Beispiel 3 in 8 ml $CF_3COOH$ wurde 2 h bei Raumtemperatur gerührt. Das Reaktionsgemisch wurde mit 16ml Toluol versetzt und eingeengt. Der Rückstand wurde in 3 ml Wasser aufgenommen, es wurden 0.35ml konzentrierte HCl (12M) zugegeben, die Lösung wurde eingeengt und der Rückstand im Vakuum getrocknet. Das gebildete Tetraamin wurde in 18ml DMF suspendiert, es wurden 1.45g BocGlyGlyONsu (4.4 mmol) und 0,6ml $Et_3N$ zugegeben. Die Reaktionslösung wurde 24 h bei Raumtemperatur gerührt, im Vakuum (0.5-1 Torr) eingeengt und der erhaltene Rückstand über eine Kieselgelsäule chromatographiert (Kieselgel 60, Merck). Elution mit $Me_2CO/MeOH/H_2O$ zwischen 30:1:1 und 15:1:1 ergab 0.94 g (77%) des reien Produktes. DC: $R_f$=0.79 in $Me_2CO/MeOH/H_2O$ 15:1:1 (Ninhydrin positiv, nach 5 min

Behandlung der Platte mit HCl-Gas)
$^1H$ NMR-Spektrum in $D_6$-DMSO (δ, ppm): 8.53 (t, 1H, $NH^{Gly3}$), 7.98 (t, 1H, $CCH_2\underline{NH}$), 7.81 (t, 1H, $NH^{Gly2}$), 6.98 (t, 1H, $NH^{Gly1}$), 3.85 (d, 2H, $J_{NH}$ 5.5 Hz, $CH_2^{Gly2}$), 3.73 (d, 2H, $J_{NH}$ 5.5 Hz, $CH_2^{Gly3}$), 3.59 (d, 2H, $J_{NH}$ 6 Hz, $CH_2^{Gly1}$), 2.69 (br. d, 2H, $J_{NH}$ 6.5 Hz, $CCH_2$), 1.38 (s, 9H, $OCMe_3$). Massenspektrum: 1239 (M+Na).

Beispiel 5

Poly-N-(2-Hydroxyethyl)-acrylamid mit 20% mol. 6SLN, (6SLN-PAA)

Zu einer Lösung von 1mg 6SLN-Gly (1.37 µM) aus Beispiel 1 in 0.1ml DMSO wurden 1.32 mg Poly(4-nitrophenylacrylat) (6.84 µM) und 7ml $Et_3N$ in 0.132 ml DMF zugegeben. Das Reaktionsgemisch wurde bei 40°C inkubiert (der Verlauf der Konjugation wurde mittels DC verfolgt: Verschwinden des Fleckes des Zucker Eduktes), nach 24 h wurden 23µl Ethanolamin zugegeben. Nach weiteren 15 h bei Raumtemperatur wurde die Reaktionslösung über eine Sephadex LH-20 Säule (1.5x25 cm, Eluent - Acetonitril/Wasser 1:1) chromatographiert. Die zuckerhaltige Fraktion wurde eingeengt, in Wasser aufgenommen, eingefroren und lyophilisiert. Es wurden 1.6 mg des Konjugat erhalten (90%).

Beispiel 6

Polyacrylsäure ($Na^+$-salt) mit 20% mol. 6SLN

Zu einer Lösung von 1 mg 6SLN-Gly (1.37 µM) in 0.1 ml of DMSO, wurde 1.32 mg Poly(4-nitrophenylacrylate) (6.84 µM) in 0.132 ml DMF und $Et_3N$ (7 µl) zugesetzt. Das Reaktionsgemisch wurde bei 40°C inkubiert (der Verlauf der Konjugation wurde mittels DC verfolgt: Verschwinden des Fleckes des Zucker Eduktes), nach 24 h wurden 230µl 0.1 M NaOH zugegeben. Nach 15 h bei Raumtemperatur wurden 15 µl 1M HCl zugegeben und das Reaktionsgemisch über eine Sephadex LH-20 Säule (1.5x25 cm, Eluent - Acetonitril/Wasser 1:1) chromatographiert. Die zuckerhaltige Fraktion wurde eingeengt, in Wasser aufgenommen, eingefroren und lyophilisiert. Es wurden 1.5 mg Konjugat erhalten (93%).

Beispiel 7

{Neu5Acα2-6Galβ1-4GlcNAc β1-NHCOCH$_2$NHCO(CH$_2$)$_4$CO(NHCH$_2$CO)$_3$NHCH$_2$-}$_4$C.

Eine Lösung von 1.2mg Tetrakis(N-tert-butyloxycarbonyl-triglycyl-amidomethyl)methan (1 µM) aus Beispiel 4 in 0.1 ml $CF_3COOH$ wurde bei Raumtemperatur gerührt. Nach 15 h wurde 1ml Toluol zugegeben und die Lösung eingeengt. Der Rückstand wurde in 0.5 ml Wasser gelöst, es wurden 5 µl konzentrierte HCl zugegeben, die Lösung eingeengt und der Rückstand im Vakuum getrocknet. Das erhaltene Tetraamin wurde in 0.2 ml DMF suspendiert, und eine Lösung von 4.9mg Neu5Acα2-6Galβ1-4GlcNAcβ1-NHCOCH$_2$NHCO(CH$_2$)$_4$COO-p-$C_6H_4NO_2$ (5 µM) in 0.3 ml DMF und 5 µl $NEt_3$ wurde zugegeben. Das Reaktionsgemisch wurde 15 h bei 20°C gerührt. Es wurden 10 µl konzentrierte Ammmoniak-Lösung zugegeben und nach einer Stunde wurde die Reaktionslösung über eine Sephadex G-50 (1.2x50 cm) gegeben (Eluent 0.05 M Ammoniak Lösung).
Die zuckerhaltige Hauptfraktion wurde eingeengt, in Wasser aufgenommen, eingefroren und lyophilisiert. Es wurden 2.9mg Konjugat erhalten(68%).

Beispiel 8

Anzucht der Influenza Viren, und Bestimmung der Inhibition der viralen Bindung an Fetuin

Die nicht adaptierten menschlichen Influenza Viren A/England/157/83 M, A/NIB/12/89 M, A/NIB/23/89 M, A/NIB/50/89 M (H1N1), A/NIB/47/89 M, A/NIB/3/90 M, A/NIB/44/90 M (H3N2), B/England/222/82 M, B/NIB/48/90 M, and B/NIB/15/89 M wurden vom National Institute for Biological Standardisation and Control (NIBSC, Potters Bar/UK) erhalten, sie waren aus klinischen Proben isoliert und wurden ausschliesslich in MDCK Zellen gezüchtet.
Die in Hühnereiern gezüchteten Influenza Viren wurden von der Viren Stammsammlung des D.I.Ivanovsky Institutes für Virology, Moskau erhalten. Diese Viren wurden in 9-10 Tage alten embryonalen Hühnereiern inkubiert.
Für die Untersuchung der viralen Bindung, wurden die virushaltigen Kulturüberstände bzw. allantoissche Flüssigkeiten durch Zentrifugation von etwaigen Zellbruchsstücken befreit. Diese Lösungen wurden ohne weitere Aufreinigung entweder sofort eingesetzt oder bis zu 4 Wochen bei 4°C gelagert.
Die Beschichtung der Mikrotiterplatten mit Rinderfetuin (Fluka, CH) wurden folgendermassen durchgeführt: Die Kavitäten einer 96er EIA Mikrotiterplatte aus Polystyrol(Flow, USA) wurden bei mit jeweils 0.1ml einer Fetuin Lösung in PBS (10 mg/ml) 2h bei 37°C inkubiert. Die Platte wurde mit einer Lösung 0.01% Tween 20 (Serva, D) in PBS und dann mit destillierten Wasser gewaschen, und entweder direkt verwenden, oder an der Luft getrocknet und bis zur Verwendung bei -20°C gelagert.
Um die Viren spezifisch an die Fetuin beschichteten Platten zu adsorbieren, wurden die viralen Kulturen mit PBS bis auf einen Hemagglutinin-Titer von 1:50-1:200 verdünnt. Die Platten wurden dann mit je 0.1ml dieser Lösung pro Kavität für 2 h bei 4°C inkubiert.
Die Inbition der Bindung des Meerrettich-Peroxidase-Fetuin Konjugates (hergestellt nach der Perjodat Aktivierungs Standardmethode) an die adsorbierten Viren wurde wie folgt bestimmt:
Die Platten wurden mit einer 0.01% Tween 20 Lösung in PBS gewaschen, dann wurden 0.1 ml einer 0.02 µM Lösung des Peroxidase-Fetuin Konjugates und gleichzeitig verschiedene Konzentration des zu testenden Inhibitors zugegeben. Nach einer Inkubationzeit von 1 h bei 4°C wurden die Platten mit einer kalten PBS/Tween Lösung gewaschen.
Die Peroxidase Aktivität wurde durch Zugabe von je 0.1 ml einer Substratlösung (0.4 mg/ml o-Phenylendiamin plus 0.02% $H_2O_2$ in 50 mM Natriumacetat Puffer, pH 5.5) bestimmt. Nach Zugabe der Substratlösung wurden die Platten 30 Minuten bei Raumtemperatur im Dunkeln inkubiert, dann wurden 0.05ml 5% $H_2SO_4$ zugegeben und die Absorbtion bei 492nm mit einem Titertek Multiscan reader (Flow, Finland) bestimmt.

Zur Bestimmung der unspezifischen Bindung, wurden auf jeder Platte einige Kavitäten ohne Zugabe von Viren inkubiert. Es wurde jeweils nur sehr geringe unspezifische Bindung gefunden ($A_{492}$ Werte von 0.05-0.2).
Zur Bestimmung der maximalen Bindung $A_{max}$ wurde für jede einzelne Platte die Bindung des Peroxidase-Fetuin-Konjugates ohne Inhibitor Zugabe gemessen.
Als weitere Kontrolle wurde jeder einzelnen Virus Stamm mit einem Peroxidase-Fetuin-Konjugat umgesetzt, welcher zuvor mit Neuraminidase aus Vibrio cholerae umgesetzt worden war: In allen Fällen verlor das Konjugat seine Affinität zu Viren, somit wurde bewiesen, daß die Wechselwirkung zwischen Konjugat und Viren durch Sialyloligosaccharide bedingt ist.

Die Bindungs Affinität wurde nach der folgenden Formel berechnet:

$$K_{aff} = \frac{C_i \times A_i \; (A_{max}\text{-}A_o)}{A_{max} \; (A_i\text{-}A_0)}$$

hierbei ist $K_{aff}$ die Dissoziationskonstante des Virus-Inhibitor Komplexes; $A_{max}$ ist die gemessene Absorbtion in Abwesenheit des Inhibitors; $C_i$ und $A_i$ sind die eingesetzte Inhibitor Konzentration und die entsprechende gemessene Absorbtion; $A_o$ ist die bei Sättigung an Inhibitor gemessene Absorption.

Die Ergebnisse der Inhibition der viralen Zelladhäsion des neuen Konjugates 6SLN-PAA aus Beispiel 5 sind in Tabelle 1 bzw. Tabelle 2 zusammengefasst.

Tabelle 2:

Inhibition der viralen Zelladhäsion von Influenza Viren durch Sialosid Inhibitoren. Die Literaturdaten wurden zusammengefasst, wobei jeweils die höchste genannte inhibitorische Aktivität aufgeführt wird. Die Aktivität wird ausgedrückt in µM Konzentration an Neu5Ac Gruppen des Inhibitors, welche eine 50% Inhibition verursachen. Verwendente Testsysteme: Inhibition der Hemagglutination (HAI), Inhibiton der Virus Adsorbtion an Erythrocyten (AI), Verringerung der viralen Vermehrung in Zellkulturen (MI), Verringerung der viralen Bindung an Fetuin (FBI). Zum Vergleich sind zuunterst die Daten der Verbindung 6SLN-PAA aus Beispiel 5 aufgeführt.

| Verbindungstyp | Test | µM Neu5Ac-Gruppen, bei50% Inhibition | Referenz |
|---|---|---|---|
| Bivalente Sialoside | HAI<br>HAI | 30 (X31)<br>180 (X31) | Glick & Knowles, 1991<br>Sabesan et al., 1992 |
| Cluster Sialoside | HAI | 20 (X31) | Roy et al., 1993 |
| Liposomale Sialoside | HAI<br>HAI | 0.02 (X31)<br>0.33 (unknown) | Kingery-Wood et al, 1992<br>Spevak et al., 1993 |
| Sialylpolymere | FBI<br>HAI<br><br>HAI<br>HAI | 0.3 (A/TX/77, A/BK/79)<br>0.2 (X31)<br><br>0.1 (X31)<br>0.2 (X31) | Matrosovich et al, 1990<br>Spaltestein & Whitesides,1991<br>Sparks et al., 1993<br>Lees et al., 1994 |

Tabelle 2: (fortgesetzt)

| Verbindungstyp | Test | µM Neu5Ac-Gruppen, bei50% Inhibition | Referenz |
|---|---|---|---|
| | MI | 0.02 (X31, H3N2) | Itoh et al., 1995 |
| | | 5 (WSN/33, H1N1) | Itoh et al., 1995 |
| | | > 400 (SG/57, H2N2) | Itoh et al., 1995 |
| | FBI | >50 (X31, H3N2) | Mochalova et al, 1994 |
| | | 0.03 (USSR/85, H3N2) | Mochalova et al, 1994 |
| | | 10 (Chile/83, H1N1) | Mochalova et al, 1994 |
| | | 10 (B/USSR/83) | Mochalova et al, 1994 |
| | MI | >8 (X31, H3N2) | Mochalova et al, 1994 |
| | | 0.2 (USSR/85, H3N2) | Mochalova et al, 1994 |
| | | 4 (Chile/83, H1N1) | Mochalova et al, 1994 |
| | | >8 (B/USSR/83) | Mochalova et al, 1994 |
| | HAI | 0.001 (X31, H3N2) | Mamen et al, 1995 |
| 6SLN-PAA wie in Beispiel 5 der vorliegenden Erfindung beschrieben | FBI | 0.02 (A/NIB/23/89M, H1N1)<br>0.01 (A/NIB/44/90M, H3N2)<br>0.02 (B/NIB/15/89M) | |

Literatur

Bovin, N.V., Byramova, N.E., Tuzikov, A.B., Matrosovich, M.N., Mochalova, L.V., and Gambaryan, A.S. (1992) Viral attachment inhibitors, Patent PCT No. PCT/US92/09745.

Byramova, N.E., Mochalova, L.V., Belyanchikov, I.M., Matrosovich, M.N., and Bovin, N.V. (1991) Synthesis of sialic acid pseudopolysaccharides by coupling of spacer-connected Neu5Ac with activated polymer. J.Carbohydr. Chem. 10, 691-700.

Fleischer, E.B., Gebala, A.E., Levey, A., Tasker, P.A. (1971). Conversion of aliphatic and alicyclic polyalcohols to the corresponding primary polyamines. J.Organic Chem., Vol. 36, No. 20, 3042-3044.

Gambaryan, A.S. and Matrosovich, M.N. (1992) A solid-phase enzyme-linked assay for influenza virus receptor-binding activity. J.Virol.Methods, 39, 111-123.

Glick, G.D., and Knowles, J.R. (1991). Molecular recognition of bivalent sialosides by influenza virus. J.Amer. Chem.Soc. 113, 4701-4703.

Gottschalk, A., Belyavin, G., Biddle, F. (1972). Glycoproteins as influenza virus haemagglutinin inhibitors and as cellular virus receptors. In "Glycoproteins. Their composition, structure and function", (A.Gottschalk, Ed.), 2-d edition, part B, pp.1082-1096. Elsevier Publishing Company, Amsterdam-London-New-York.

Itoh, M., Hetterich, P., Isecke, R., Brossmer, R., and Klenk, H.-D. (1995). Supression of influenza virus infection by an N-thioacetylneuraminic acid acrylamide copolymer resistant to neuraminidase. Virology 212, 340-347 (1995).

Kingery-Wood, J.E., Williams, K.W., Sigal, G.B., and Whitesides, G.M., (1992). The agglutination of erythrocytes by influenza virus is strongly inhibited by liposomes incorporating an analog of sialyl gangliosides. J.Amer.Chem. Soc. 114, 7303-7305.

Krizanova, O., and Ratnova, V. (1969). Serum inhibitors of myxoviruses. Curr.Top.Microbiol.Immunol. 47, 125-151.

Lees, W.J., Spaltenstein, A., Kingery-Wood, J.E., and Whitesides, G.M. (1994). Polyacrylamides bearing pendant a-sialoside groups strongly inhibit agglutination of erythrocytes by influenza A virus: multivalency and steric stabilization of particulate biological systems. J.Med.Chem. 37, 3419-3433.

Likhosherstov, L.M., Novikova, O.S., Derevitskaja, V.A., Kochetkov, N.K. (1986). A new simple synthesis of amino sugar b-D-glycosylamines. Carbohydrate Research, 146, C1-C5.

Mammen, M., Dahmann, G., Whitesides, G.M. (1995) Effective inhibitors of hemagglutination by influenza virus synthesized from polymers having active ester groups. Insight into mechanism of inhibition. J.Med.Chem., 38, 4179-4190.

Manger, I.D., Rademacher, T.W., Dwek, R.A. (1992). 1-N-Glycyl b-oligosaccharide derivatives as stable intermediates for the formation of glycoconjugate probes. Biochemistry, 31, 10724-10732.

Matrosovich, M.N., Mochalova, L.V., Marinina, V.P., Byramova, N.E., and Bovin, N.V. (1990). Synthetic polymeric sialoside inhibitors of the influenza virus receptor-binding activity. FEBS Letters 272, 209-212.

Matrosovich M.N., Gambaryan A.S., Tuzikov A.B., Byramova N.E., Mochalova L.V., Golbraikh A.A., Shenderovich M.D., Finne J., and Bovin, N.V. (1993). Probing of the receptor-binding sites of the H1 and H3 influenza A and influenza B virus hemagglutinins by synthetic and natural sialosides. Virology 196, 111-121.

Mochalova, L.V., Tuzikov, A.B., Marinina, V.P., Gambaryan, A.S., Byramova, N.E., Bovin, N.V., and Matrosovich, M.N. (1994) Synthetic polymeric inhibitors of influenza virus receptor-binding activity suppress virus replication. Antiviral Research 23, 179-190.

Robertson, J.S. (1993). Clinical influenza virus and the embryonated hen's egg. Rev.Med.Virol. 3, 97-106.

Roy, R., and Laferriere, C.A. (1988). Synthesis of antigenic copolymers of N-acetyl-neuraminic acid and their binding to wheat germ agglutinin and antibodies. Carbohydr. Res. 177, c1-c4.

Roy, R., Zanini, D., Meunier, S.J., and Romanowska, A. (1993). Solid-phase synthesis of dendritic sialoside inhibitors of influenza A virus hemagglutinin. J.Chem.Soc., Chem. Commun. 1993, 1869-1872.

Sabesan, S., Duus, J.O., Neira, S., Domaille, P., Kelm, S., Paulson, J.C., and Bock, K. (1992). Cluster sialoside inhibitors for influenza virus: synthesis, NMR, and biological studies. J.Amer.Chem.Soc. 114, 8363-8375.

Sauter, N.K., Hanson, J.E., Glick, G.D., Brown, J.H., Crowther, R.L, Park, S.-J., Skehel, J.J., and Wiley, D. C. (1992) Binding of influenza virus hemagglutinin to analogs of its cell-surface receptor, sialic acid: analysis by proton nuclear magnetic resonance spectroscopy and X-ray crystallography. Biochemistry 31, 9609-9621.

Spaltenstein, A., and Whitesides, G.M. (1991). Polyacryamides bearing pendant a-sialoside groups strongly inhibit agglutination of erythrocytes by influenza virus. J.Amer.Chem.Soc. 113, 686-687.

Spevak, W., Nagy, J.O., Charych, D.H., Schaefer, M.E., Gilbert, J.H., and Bednarski, M.D. (1993). Polymerized liposomes containing C-glycosides of sialic acid: potent inhibitors of influenza virus in vitro infectivity. J.Amer.Chem. Soc. 115,1146-1147.

Toogood, P.L., Galliker, P.K., Glick, G.D. and Knowles, J.R. (1991) Monovalent sialosides that bind tightly to influenza A virus. J.Med.Chem. 34, 3138-3140.

Unverzagt, C., Kelm, S., Paulson, J. C., (1994) Chemical and enzymatic synthesis of multivalent sialoglycopeptides, Carbohydrate Research 251, 285-301. Watowich, S.J., Skehel, J.J., and Wiley, D.C. (1994) Crystal structures of influenza virus hemagglutinin in complex with high-affinity receptor analogs. Structure 2, 719-731.

Weinhold, E.G. and Knowles, J.R. (1992) Design and evaluation of a tightly binding fluorescent ligand for influenza A hemagglutinin. J.Am.Chem.Soc. 114, 9270-9275.

Wiley, D.C. and Skehel, J.J. (1987) The structure and function of the hemagglutinin membrane glycoprotein of influenza virus. Ann.Rev.Biochem. 56, 365-394.

## Patentansprüche

1. Verbindungen der allgemeinen Formel,

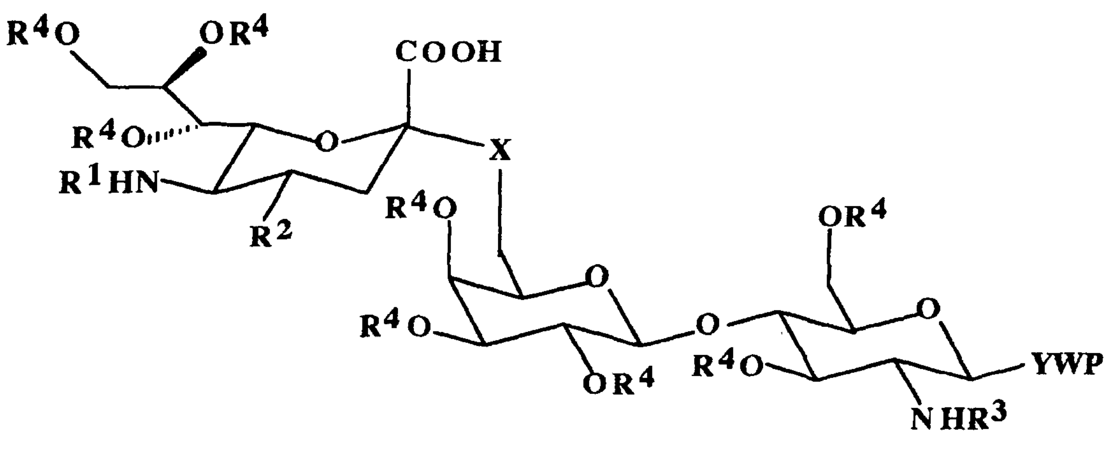

worin $R^1$ - eine Acyl oder Thioacylgruppe bedeutet;

$R^2$ - H, Hydroxyl, Z-Alkyl, substituiertes Z-Alkyl, Z-Aryl, substituiertes Z-Aryl bedeutet, und Z -O, S oder NH entspricht;

$R^3$ - eine Acyl oder Thioacylgruppe bedeutet;

$R^4$ - H oder Acyl darstellt;

X - O, S oder ein lineares $C_1$-$C_4$ Alkyl bedeutet;
Y - NH, O, S, $CH_2$, oder ein Zucker ist;
W - ein bifunktioneller Spacer ist;
P - ein multivalenter Träger bestehend aus einem der nachfolgenden Stoffe ist:

Polyacrylat, Polyacrylamid, N-substituiertes Polyacrylamid, Metacrylamid, N-substituiertes Metacrylamid, Polyacrylsäure, Polycarbonat, Polyester, Polyamid, Polyanhydrid, Polyiminocarbonat, Polyorthoester, Polydioxanon, Polyphosphazen, Polyhydroxycarbonsäure, Polyaminosäure, Polysaccharid, Protein, Dextran, Chitosan, Glucan, Liposom, Micropartikel.

**2.** Verbindungen der Formel I gemäß Anspruch 1, **dadurch gekennzeichnet, daß** der multivalente Träger $(-A_n-NHCH_2)_4$ C darstellt, worin n=3 und A eine neutrale oder negativ geladene Aminosäure ist.

**3.** Verbindungen der Formel I gemäß den Ansprüchen 1 und 2 worin P mit Biotin, einem Farbstoff, einem Fluoreszenzfarbstoff oder einem radioaktiven Marker markiert ist.

**4.** Verwendung der Verbindungen der Formel I gemäß den Ansprüchen 1 und 2 zur Bindung an Viren.

**5.** Verwendung der Verbindungen der Formel I gemäß den Ansprüchen 1 und 2 zur Bindung an Influenza Viren.

**6.** Verwendung der Verbindungen der Formel I gemäß den Ansprüchen 1 und 2 zur Bindung an menschliche Influenza Viren.

**7.** Verwendung der Verbindungen der Formel I gemäß dem Anspruch 3 als Komponenten von Testsystemen zum Screenen von Inhibitoren der viralen Zelladhäsion.

**8.** Verwendung der Verbindungen der Formel I gemäß den Ansprüchen 1 und 2 zur Bindung an Hemagglutinin von Influenza Viren.

**9.** Verwendung der Verbindungen der Formel I gemäß den Ansprüchen 1 und 2 als Inhibitoren der viralen Zelladhäsion an natürliche Rezeptoren.

**10.** Verwendung der Verbindungen der Formel I gemäß den Ansprüchen 4-9 in Kombination mit Neuraminidase-Inhibitoren.

**11.** Verwendung der Verbindungen der Formel I gemäß den Ansprüchen 1 und 2 zur Herstellung von Arzneimitteln für eine prophylaktische oder therapeutische Behandlung viraler Infektionen.

**12.** Verwendung der Verbindungen der Formel I gemäß den Ansprüchen 1 und 2 zur Herstellung von Arzneimitteln für eine prophylaktische oder therapeutische Behandlung viraler Infektionen in Kombination mit Neuraminidase-Inhibitoren.

## Claims

**1.** Compositions of the general Formula I

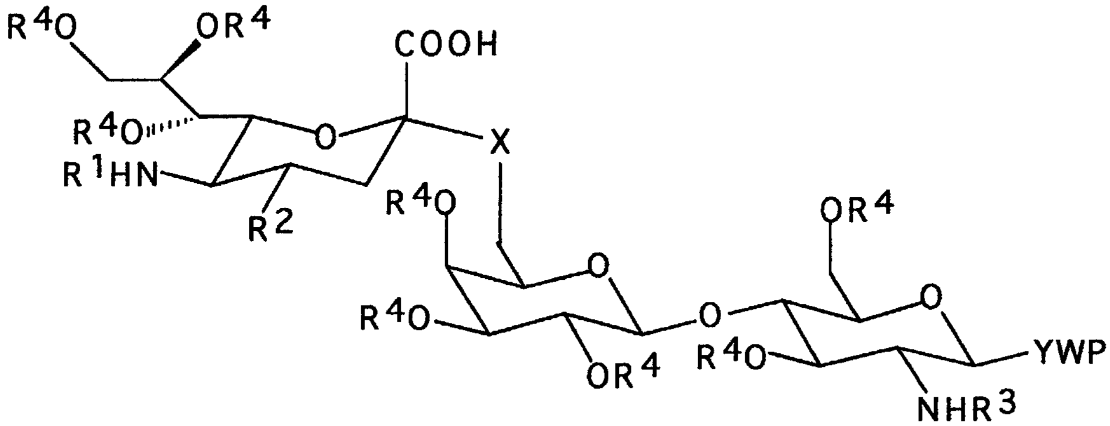

where $R^1$ means an acyl group or a thioacyl group;

$R^2$ - is selected from the group consisting of H, hydroxyl, Z-alkyl, substituted Z-alkyl, Z-aryl, substituted Z-aryl, and Z corresponds to O, S or NH;

$R^3$ - means an acyl group or a thioacyl group;

$R^4$ - represents H or acyl;

X - is selected from the group consisting of O, S or a linear $C_1$-$C_4$ alkyl;
Y - is selected from the group consisting of NH, O, S, $CH_2$ or a sugar;
W - is a bifunctional spacer;
P - is a multivalent carrier consisting of one of the following substances:

polyacrylate, polyacrylamide, N-substituted polyacrylamide, methacrylamide, N-substituted methacrylamide, polyacryl acid, polycarbonate, polyester, polyamide, polyanhydride, polyiminocarbonate, polyorthoester, polydioxanon, polyphosphazen, polyhydroxycarbon acid, polyamino acid, polysaccharide, protein, dextran, chitosan, glucan, liposome, microparticle.

**2.** Compositions of Formula I described in claim 1, wherein the multivalent carrier is $(-A_n-NHCH_2)_4C$, wherein A is a neutral or negatively charged amino acid.

**3.** Compositions of Formula I described in claim 1 and claim 2 wherein P is labeled with biotin, a dye, a fluorescent dye or a radioactive marker.

**4.** Use of compositions of Formula I described in claim 1 and claim 2 for binding to viruses.

**5.** Use of compositions of Formula I described in claim 1 and claim 2 for binding to influenza viruses.

**6.** Use of compositions of Formula I described in claim 1 and claim 2 for binding to human influenza viruses.

**7.** Use of compositions Formula I described in claim 3 as components of test systems for screening virus to cell adhesion inhibitors.

**8.** Use of compositions of Formula I described in claim 1 and claim 2 for binding to influenza virus hemagglutinin.

**9.** Use of compositions of Formula I described in claim 1 and claim 2 as inhibitors of viral cell adhesion to natural receptors.

**10.** Use of compositions of Formula I in accordance with claims 4 to 9 in combination with neuraminidase inhibitors.

**11.** Use of compositions of Formula I described in claim 1 and claim 2 for the preparation of medicinal products for prophylactic or therapeutic treatment of viral infections.

**12.** Use of compositions of Formula I described in claim 1 and claim 2 for the preparation of medicinal products for prophylactic or therapeutic treatment of viral infections in combination with neuraminidase inhibitors.

**Revendications**

**1.** Des composés dont la formule générale est la suivante :

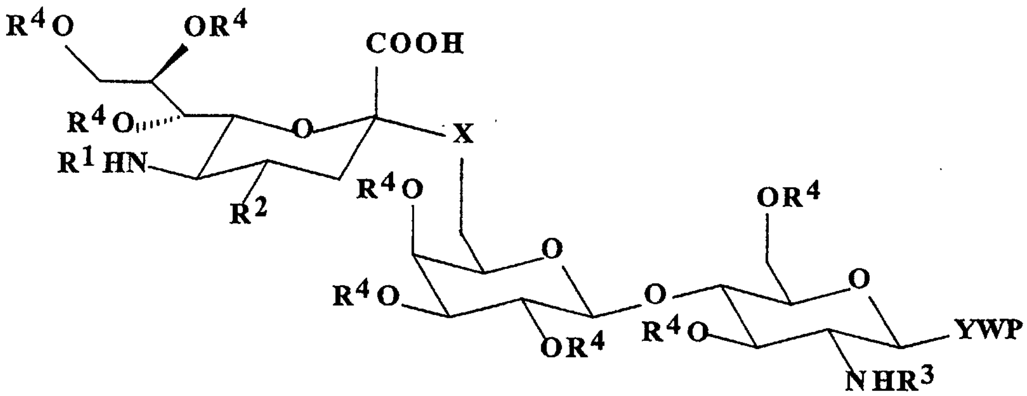

Dans laquelle le groupe $R^1$ représente un groupe acyle ou un groupe thioacyle ;
$R^2$ - est sélectionné du groupe constitué de H, hydroxyle, Z-alkyle, Z-alkyle substitué, Z-aryle, Z-aryle substitué, et Z correspond à O, S ou NH ; $R^3$ - représente un groupe acyle ou un groupe thioacyle ; $R^4$ - représente H ou acyle ;
X - est sélectionné du groupe constitué de O, S ou une chaîne alkyle linéaire $C_1$-$C_4$ ;
Y - est sélectionné du groupe constitué de NH, O, S, $CH_2$ ou un sucre ;
W - est un bras espaceur bifonctionnel ;
P - est un porteur multivalent constitué d'une des substances suivantes :

polyacrylate, polyacrylamide, polyacrylamide N-substitué, méthacrylamide, méthacrylamide N-substitué, acide polyacrylique, polycarbonate, polyester, polyamide, polyanhydride, polyiminocarbonate, polyorthoester, polydioxanone, polyphosphazène, acide polyhydroxycarbonique, polyaminoacide, polysaccharide, protéine, dextran, chitosan, glucan, liposome, microparticle.

**2.** Des composés selon la formule I de la revendication 1, dans laquelle le porteur multivalent est $(A_n\text{-}NHCH_2)_4C$, dans laquelle n=3 et A est un acide aminé neutre ou chargé.

**3.** Des composés selon la formule I de la revendication 1 et de la revendication 2 dans laquelle P est marqué avec une biotine, une coloration, un marqueur fluorescent ou un marqueur radioactif.

**4.** Utilisation des composés selon la formule I de la revendication 1 et de la revendication 2 pour des liaisons à des virus.

**5.** Utilisation des composés selon la formule I de la revendication 1 et de la revendication 2 pour des liaisons aux virus de la grippe.

**6.** Utilisation des composés selon la formule I de la revendication 1 et de la revendication 2 pour des liaisons aux virus de la grippe humaine.

**7.** Utilisation des composés selon la revendication 3 comme composantes de systèmes tests pour le screening d'inhibiteurs de l'adhésion virale aux cellules.

**8.** Utilisation des composés selon la formule I de la revendication 1 et de la revendication 2 pour des liaisons à l'hémagglutinine du virus de la grippe.

**9.** Utilisation des composés selon la formule I de la revendication 1 et de la revendication 2 comme inhibiteurs de l'adhésion virale aux cellules de récepteurs naturels.

**10.** Utilisation des composés en accord avec les revendications 4 à 9 en combinaison avec des inhibiteurs de la neuraminidase.

**11.** Utilisation des composés selon la formule I de la revendication 1 et de la revendication 2 pour la préparation de produits médicinaux pour des traitements prophylactiques ou thérapeutiques d'infections virales.

**12.** Utilisation des composés selon la formule I de la revendication 1 ou de la revendication 2 pour la préparation de produits médicinaux pour des traitements prophylactiques ou thérapeutiques d'infections virales en combinaison avec des inhibiteurs de la neuraminidase.